# EUROPEAN PATENT SPECIFICATION

(11) **EP 1 177 788 B1**
(45) Date of publication and mention of the grant of the patent: **14.11.2007**
(21) Application number: 01306472.0
(22) Date of filing: 27.07.2001
(51) Int. Cl.: A61K 31/138, A61K 9/36

(54) **Pharmaceutical composition of fluoxetin in coated dispersible tablets and process for its manufacture**
Fluoxetin-Arzneimittel in Form überzogener dispergierbarer Tabletten sowie Verfahren zu dessen Herstellung
Composition pharmaceutique à base de fluoxétine sous forme de comprimés enrobés dispersibles et son procédé de fabrication

(30) Priority: 01.08.2000 ES 200001952
(43) Date of publication of application: 06.02.2002
(73) Proprietor: Laboratorios Cinfa,S.A., 31620 Huarte-Pamplona, Navarra (ES)
(72) Inventor: Ursua Sesma, Angel, 31620 Huarte, Pamplona, Navarra (ES); Martinez de Narvajas Alcalde, Felix, 31620 Huarte, Pamplona, Navarra (ES); Yanez-Sedeno Congosto, Carlos, 31620 Huarte, Pamplona, Navarra (ES); Larumbe Oroz, Juan Pedro, 31620 Huarte, Pamplona, Navarra (ES)
(74) Representative: March, Gary Clifford

(56) References cited:
- DE-A- 4 122 039
- DE-A- 4 141 268
- FR-A- 2 288 514
- US-A- 5 100 675
- US-A- 5 736 159
- US-A- 5 985 322

## Description

The invention relates to a dispersible tablet of fluoxetin improved so as to be provided with a water-soluble laminar coating designed for administration by oral means, and also relates to a process for its manufacture.

In particular, this invention relates to a new tablet which makes it possible to administer a therapeutically effective quantity of fluoxetin hydrochloride for rapid bioavailability without causing the persistent bitter effect which in the long term can make satisfactory continuation or compliance with the treatment on the part of the patient difficult.

Fluoxetin or "N-methyl-3-(p-trifluoromethyl-phenoxy)-3-phenylpropylamine" is an antidepressant agent whose action appears to be based on its capacity to selectively inhibit the capture of serotonin by neurones of the central nervous system. Fluoxetin is especially indicated for the treatment of depression and associated anxiety, as well as for the treatment of nervous bulimia and obsessive heightened compulsive disturbances.

Forms which can be administered by oral means are more particularly suitable for out-patient treatment, in which the patient must comply with the treatment prescribed. The pharmaceutical forms hitherto available for the administration of fluoxetin hydrochloride are capsules, solution, sachets and dispersible tablets.

In fact Spanish patent no. 0433720 by Lilly describes a process for the preparation of "3-aryloxy-3-phenylpropylamines" and the acid addition salts of the same which are useful as psychotropic agents, especially antidepressants. Included within the scope of this patent are pharmaceutically acceptable salts of the amine bases which are formed with non-toxic inorganic and organic acids. In this patent different methods for synthesising and obtaining these bases and their corresponding salts, among which are included the addition salts of an acid, such as hydrochlorides, are described.

Likewise this patent describes possible forms for the administration of the substances to human beings, indicating that for oral administration the salt can be mixed with normal pharmaceutical excipients and placed in rigid gelatin capsules. In the same way the compound can be mixed with starch, agglutinating agents, etc., to be formed into tablets, which can be grooved to facilitate administration in subdivided doses. For parenteral formulation soluble salts of these compounds which dissolve in an isotonic solution for intramuscular, intravenous or subcutaneous administration are used.

United States patent 4,061,747 by Aktiebolaget Leo describes different pharmaceutical forms used for administration of the antidepressant "lofepramine". The pharmaceutical forms described in this patent include tablets, pills, dispersible powders, granules and capsules.

In the article by Trudi Martin published in volume 7 of the publication "New-Zealand Pharmacy" of 1987, the title of which is "dispersible tablets as an alternative to mixtures", the dispersible behaviour of a series of tablets is described, and among these reference is made to five antidepressants (amoxapine, amitriptiline, imipramine, mianserine and trimipramine), of which amoxapine and trimipramine clearly act as dispersible tablets in comparison with the other three.

PCT application WO-A-93 09769 by Sepracor Inc. describes a pharmaceutical composition which is appropriate for the manufacture of dispersible tablets through a direct compression process, as described on page 23 lines 24 to 31. Likewise this application discloses that the pharmaceutical composition has fluoxetine or an acid addition salt thereof as the active ingredient, as described on page 30 starting from line 10. This pharmaceutical composition, in which the active ingredient is fluoxetin, contains appropriate coadjuvants and excipients as described on page 22 lines 20 to 24.

Like the Sepracor patent, Spanish patent number 9401593 by Lilly describes a pharmaceutical formulation of fluoxetin in dispersible form in which the tablets are obtained by the already mentioned process of direct compression, describing a specific formulation in which the active principle, comprising fluoxetin or its acid addition salts, appears in a quantity of between 4% and 7.5% by weight with respect to the total for the formulation, this active ingredient being supplemented by appropriate excipients and coadjuvants.

Some disadvantages associated with the abovementioned forms are described below.

The use of capsules has limitations and disadvantages relating to:
- their application to the patient, which may be limited given that some patients may have problems with swallowing the capsules, especially children and old persons, who also may be incapable of swallowing the capsules, and
- its pharmaceutical form per se, as it only permits a single dose.

The administration of fluoxetin hydrochloride in solution form suffers from the following disadvantages:
- a dose of the active ingredient which requires the use of measuring instruments, which are not normally precise,
- limited possibility for use in diabetic patients given that these have to take appropriate precautions on account of the saccharose content in the formulation,
- the existence of a risk of accidental overdose through uncontrolled consumption, especially in children, and
- little ease of handling and transport due to the volume required, which is associated with a certain risk of therapeutic non-compliance which may give rise to a reduction in the efficacy of the treatment.

On the other hand the treatment of depression requires the constant and prolonged consumption (up to 6 months) of effective doses of antidepressants such as fluoxetin. Fluoxetin, especially in the form of the hydrochloride, has a strongly bitter and disagreeable taste, as a result of which its administration in the form of solution, sachets or even in the form of dispersible tablets dispersed in water gives rise to acceptance problems in patients who are obliged to ingest it over prolonged periods of time. These problems can give rise to non-compliance with the treatment which considerably reduces the efficacy of treatment. An additional disadvantage in respect of sachets and dispersible tablets is the need to disperse them in a volume of water when preparing doses, which means that the entire volume of liquid in which it is dispersed has to be ingested, which can give rise to the problem of the ingestion of a volume of liquid having a bitter taste, which induces the patient not to take the entire dose.

Another disadvantage relating to dispersible tablets is that they should disaggregate in less than three minutes in water between 19°C and 21°C and produce a uniform dispersion in water, in accordance with the uniformity of dispersion tests described in the British Pharmacopoeia, vol II, 1988, and in the European Pharmacopoeia 3rd Edition, this test only considering that the dispersion produced after two tablets have been dispersed in 100 ml of water should after shaking pass through a sieve having a nominal mesh size of 710 microns. This test does not take into account that not all the fluoxetin in the dispersion may dissolve and may be retained in the excipients which sediment out, and that the patient may not take them on account of their disagreeable aspect and their bitter taste, which again has an effect on non-compliance with the treatment.

US-A-5,985,322 relates to an enteric fluoxetine formulation and discloses a pharmaceutical composition according to the pre-characterising portion of claim 1.

Aspects of the present invention are defined in the appended independent claims, to which reference should now be made. Embodiments of the invention are defined in the appended dependent claims, to which reference should also now be made.

As a consequence, this invention relates to a new pharmaceutical formulation which contains fluoxetin such as its hydrochloride as the active ingredient and which is suitable for the preparation of dispersible tablets which have the special feature that they possess a water-soluble film coating. This invention also relates to the said coated dispersible tablets which contain fluoxetin hydrochloride as an active ingredient and a process for their manufacture.

One advantage of coated dispersible tablets in comparison with dispersible tablets must be pointed out, and this is that the former allow the possibility that the tablet can be administered as such, directly by oral means, in such a way that prior dispersion in water and therefore possible losses of dose, the bitter taste and the disadvantage associated with non-compliance with treatment can be avoided. The laminar coating is water-soluble, is temporarily but sufficiently effective to prevent disintegration of the core and premature release of the active ingredient within the mouth, and in addition to this the coating has the effect that the surface of the tablet has a better appearance, being smoother and therefore easier to swallow. The tablet disaggregates immediately on reaching the stomach. The coating also improves the behaviour of the dispersible tablets in the course of pharmaceutical manufacture, assists the process of primary packaging, as they flow better, and prevents possible contamination and soiling due to dust.

This invention relates to the provision of coated dispersible tablets giving rise to an improvement in this pharmaceutical form designed for administration by oral means which is characterised in that it comprises:
- a nucleus or core of fluoxetin such as its hydrochloride, and appropriate excipients for the preparation of the said core through a specific process which are appropriate for the stability and release of this active ingredient,
- a water-soluble laminar coating of temporary but sufficient effectiveness to preserve the core from disintegration and premature release of the active ingredient in the mouth although it permits release in the stomach.

As described in this description the invention relates to a new design of coated dispersible tablet for the administration of a therapeutically effective quantity of fluoxetin such as its hydrochloride by oral means which does not give rise to any persistent bitter taste in the oral cavity giving rise to an attitude of repulsion on the part of patients when in use. These tablets have appreciable physical strength and stability, which makes them more suitable for primary packaging under storage conditions.

In the wider sense of the invention the tablets are characterised in that their nuclei contain between 8.4% and 8.5% of active ingredient, the remainder preferably being suitable quantities of disaggregating agents, diluents, lubricants, adhesion-preventing agents, sweeteners and flavourings; the said cores are coated with a film which preferably represents around 1% by weight of the tablet, in such a way that the composition of the film comprises a water-soluble film-forming agent and a plasticiser.

The quality of the remaining ingredients preferably should be as high as possible to utilize a process of direct compression which permits adequate disaggregation of the tablet.

More preferably the composition of a nucleus or core comprises:
- as active ingredient, fluoxetin hydrochloride present as 8.43% by weight with respect to the total weight of the core in the formulation. The fluoxetin must be of micronised quality to encourage and ensure a homogeneous and intimate distribution of the mixture and ensure its rapid dissolution and absorption. The term 'micronised' as used herein in relation to the fluoxetin active ingredient means that 100% of the particles of the raw material must have a size lower than 37 micron (i.e. 100%<37µ) and furthermore at least 50% of the particles must be smaller than 10micron (i.e. 50% <10µ).

Specifically, the particle size distribution is preferably as follows: d (0.1) not less than 5 microns; d (0.5) between 33 and 55 microns; d (0.9) not less than 180 microns. The behaviour in the formula of the micronised starting material is entirely different from that of the unmicronised starting material.
- the disaggregating agent is preferably sodium croscarmellose as 11.31% by weight with respect to the weight of the core, it is a crospovidone, an insoluble cross-linked polyvinylpyrrolidone, specifically the commercial product "Kollidon CL." from the Rohm company is suitable and preferred. This polymer produces disaggregation on swelling through the effect of water or in the stomach. In this way it encourages rapid dissolution of the active ingredient in the medium into which it is released.
- microcrystalline cellulose as 45.60% by weight with respect to the weight of the nucleus is preferably used as diluent, this excipient facilitates fluidity of the mixture and its direct compression and provides the core with strength for subsequent coating. This excipient performs a double function, also acting as an agglomerating agent which also encourages rapid disaggregation. Specifically commercial product "Avicel PH 102" from the company FMC Foret is suitable and preferred, which encourages fluidity of the mixture due to its relatively large particle size, which facilitates direct compression of the fine powder,
- also preferably included is another excipient having diluting and aggregating properties, this is pregelatinised maize starch as 16.96% by weight with respect to the weight of the core. The commercial product used for this excipient is most preferably a one-to-one mixture of the commercial products "Sepistab ST 200" from the Seppic company and "Starch 1500 LM" from the Colorcon company is used, even though they are the same compound according to the commercial description, it has been shown that they have different characteristics on account of differences in particle size, and in the process through which the gelatin is applied. The use of "Sepistab ST 200" in the preferred percentage improves dispersion of the tablet, but has the disadvantage of poorer fluidity in the powder which compromises the compression process, with the occurrence of slight sticking to the punch logos. The use of "Starch 1500 LM" improves the fluidity of the powder and the phenomenon of sticking to the logos does not occur, but it retards dispersion. To make use of the advantages of both, a formulation has been developed which includes a mixture of the two in a proportion of one to one, in the percentage indicated previously, evidence having been provided in the examples for the use of both of them separately, as well as the mixture described above.

"Starch 1500 LM" pregelatinised maize starch is a partly pregelatinised starch in that the structure of the maize starch is altered to a partly pregelatinised form. In this form the link between some of the glucoses in the maize starch (amylose and amylopeptin) is broken and it has approximately the following composition: 15% amylopeptin, 5% amylose and 80% physically unaltered maize starch. This composition together with the morphology of the particles obtained in the manufacturing process confer the properties which distinguish this disintegrating, diluent, binding or agglutinant excipient and lubricant for direct compression.

"Sepistab ST 200" pregelatinised maize starch has a mean granule particle size of 200 microns. Through microscopic analysis it is seen that the product forms aggregates of regular spherical rough starch granules joined together by point-to-point contact. The tap density is 0.7 g/ml. As a result of its mean particle size and its morphology it encourages flow characteristics and compressibility and subsequently disaggregation.

Both types of pregelatinised starch are preferably used in order to make use of the advantages of each in combined form.
- stearyl sodium fumarate as 2.6% by weight with respect to the weight of the core is preferably used as a lubricant, this excipient increases slip in the powder and encourages uniform filling of the dye in the compressor, yielding tablets with very little variation in weight. The commercial product preferred is "Pruv". This excipient does not interfere with the solubility of the active ingredient as may be the case with other conventional salts of stearic acid which are also used as lubricants.
- colloidal silicon dioxide as 1.51% by weight with respect to the weight of the core is preferably used as an anti-adhesion agent, this excipient is also lubricating and encourages the flow of powder, as well as being an absorbent for the moisture which delays the appearance of degradation in the finished product. The commercial product preferably used is "Aerosil 200".
- sodium saccharine as 4.52% by weight with respect to the weight of the core is preferably used as an artificial sweetener, this excipient acts to mask the bitter taste if the coated dispersible tablet is dispersed in water for administration.
- other artificial sweeteners optionally included in the formulation when the tablet is dispersed in water may be D-mannitol, commercial product "C Mannidex 16700", or xylitol, either of these preferably being used at 7.54% by weight with respect to the weight of the core. In the formulation xylitol was more preferred on account of its more refreshing taste. Both perform equally in the formulation, but the latter helps to mask the taste even more. In fact xylitol is the polyol having the greatest sweetening power (approximately twice that of mannitol), the perception of sweetness is faster, it has a greater refreshing power, does not leave any residual taste and is more soluble than mannitol. The xylitol preferred is a granulate having the commercial description of "Xilitab 300" from the Xirofin company. This xylitol has a concentration of 100% and a mean particle size of 150 microns.

Mint flavour, preferably at 1.51% by weight with respect to the weight of the core is also preferred as a flavouring.

A preferred composition of the coating is as described below:
- a water-soluble film-forming agent such as hydroxypropylmethyl cellulose, having a viscosity of between 2.5 and 17.5 mPas.
- a plasticiser such as polyethylene glycol 6000 of average molecular weight.

The preferred mixture comprises an aqueous selection of hydroxypropylmethylcellulose and polyethyleneglycol 6000. This mixture is preferably applied as a spray onto the cores until they show an average weight gain of 1%. On the other hand the laminar coating produced by the film under the conditions of the invention not only reproduces but also improves logos on cores, indented or in relief, indicating the active ingredient and its dose, which can be obscured, eroded or erased or in any event rendered illegible in the course of coating operations with inappropriate compositions.

A preferred process for the manufacture of coated dispersible fluoxetin hydrochloride tablets is described in detail below by way of non-limiting example, and this process was used for all non-limiting formulation examples described subsequently.

First of all, all the components of the formulation are sieved through a 0.6 mm mesh sieve.

After this the following partial mixtures are made using a "V" mixer in each case, as well as for final mixing:

Fluoxetin hydrochloride is mixed with 70% of the total by weight of the microcrystalline cellulose for 10 minutes.

Separately the mint flavour is mixed with the remainder of the microcrystalline cellulose, again for 10 minutes, in order to achieve an adequate dilution.

The sodium croscarmellose, the pregelatinised starch, the sodium saccharine, the colloidal silicon dioxide and the xylitol are also mixed separately for 10 minutes.

Finally these three partial mixtures are mixed together and the sodium stearyl fumarate is added, mixing for 10 minutes. This partial mixtures system is most preferred to ensure that dispersion of the dispersible tablet is as appropriate as possible.

The final moisture content of the mixture must lie between 3 and 4%. This is an essential parameter, both for tablet formation and to ensure stability after application of the coating.

The homogeneity of the mixture is preferably determined to check that the active ingredient is equally distributed throughout the mixture.

The core of the tablet is formed through the technique of direct compression of the mixture, and if the powder flows well and the compressibility is satisfactory hardness within the preferred limits is achieved. A rotary tabletting machine with appropriate cylindrical punches to produce tablets having a theoretical mass of 265.36 mg is used to prepare these cores.

The laminar coating is applied to the cores in a perforated rotatory pump coating machine using a suspension for forming the laminar coating at a temperature between 35°C and 45°C and under conditions such that a uniform laminar coating of the cores is achieved through an increase in weight of 1%.

Once the dispersible tablets with a laminar coating have been prepared they are packed in aluminium/aluminium blisters to ensure their stability. This presentation in an aluminium/aluminium packaging material is also most preferred for satisfactory product stability.

### EXAMPLES

Coated dispersible tablet cores were prepared from the following pharmaceutical formulation.

The process of manufacture used is the same for all the examples. The first two examples demonstrate the effect of the quality of the pregelatinised starch excipient in the formulation, based on the commercial product, and conclude with a proposal for a one-to-one mix of two commercial products of this excipient; in the second example another artificial sweetener is used, this is xylitol, replacing mannitol. These two batches were used to develop the coating which has to be applied, without this interfering with other conclusions about the mixture, as shown by the results of the tests performed on the cores in both cases. The next two examples demonstrate the effectiveness of the proposed pregelatinised starch mixture, and point out the differences due to the artificial sweeteners mannitol or xylitol, as well as demonstrating the effectiveness of the application of the aqueous coating developed.

Analytical data are provided for each of the examples mentioned above.

### Example 1:

Dispersible tablet cores were prepared from the following pharmaceutical formulation using the process of manufacture described above:

| **COMPONENTS** | **CORE** | **%** | **TOTAL BATCH** |
|---|---|---|---|
| Colloidal silicon dioxide (Aerosil 200) | 4.0 mg | 1.51% | 76.00 g |
| Pregelatinised starch (Sepistab) | 45.0 mg | 16.96% | 855.00 g |
| Mint flavour 90351-51 | 4.0 mg | 1.51% | 76.00 g |
| Microcrystalline cellulose (Avicel PH 102) | 121.0 mg | 45.60% | 2,299.00 g |
| Fluoxetin hydrochloride | 22.36 mg | 8.43% | 424.84 g |
| Crospovidone (Kollidon CL) | 30.0 mg | 11.31% | 570.00 g |
| D-mannitol (C Mannidex 16700) | 20.0 mg | 7.54% | 380.00 g |
| Sodium stearyl fumarate (Pruv) | 7.0 mg | 2.64% | 133.00 g |
| Sodium saccharine | 12.0 mg | 4.52% | 228.00 g |
| TOTAL | 265.36 mg | 100.00% | 5,041.84 g |

The powder flowed with some problems and sticking to the logos in the punch occurred with some tablets, but on the other hand dispersion of the core was very good, with virtually instantaneous breakdown of the core in water between 19°C and 21°C. The compressibility of the core was good, achieving the hardness levels desired.

Coated dispersible tablets having the following characteristics were obtained:
Biconvex cylindrical white tablets 9.2 mm in diameter.
Theoretical weight of the core in an individual tablet: 265.36 mg ± 5%
Hardness stroke (maximum-minimum): (93-60 N).
Thickness: 4.08 mm.
Moisture content: 3.71%
Disaggregation between: 20 and 37 seconds (6 tablets).

As a conclusion it is suggested that a formulation in which the "Sepistab" is replaced by "Starch 1500" should be prepared in order to investigate the effect of these on the mixture.

### Example 2:

Dispersible tablet cores were prepared from the following pharmaceutical formulation in accordance with the process of manufacture described above, replacing the commercial pregelatinised starch (Sepistab) by pregelatinised starch (Starch 1500) and replacing the mannitol by xylitol:

| **COMPONENTS** | **CORE** | **%** | **TOTAL BATCH** |
|---|---|---|---|
| Colloidal silicon dioxide (Acrosil 200) | 4.0 mg | 1.51% | 76.00 g |
| Pregelatinised starch (Starch 1500) | 45.0 mg | 16.96% | 855.00 g |
| Mint flavour 90351-51 | 4.0 mg | 1.51% | 76.00 g |
| Microcrystalline cellulose | 121.0 mg | 45.60% | 2,299.00 g |
| Fluoxetin hydrochloride | 22.36 mg | 8.43% | 424.84 g |
| Crospovidone (Kollidon CL) | 30.0 mg | 11.31% | 570.00 g |
| Xylitol | 20.0 mg | 7.54% | 380.00 g |
| Sodium stearyl fumarate (Pruv) | 7.0 mg | 2.64% | 133.00 g |
| Sodium saccharine | 12.0 mg | 4.52% | 228.00 g |
| TOTAL | 265.36 mg | 100.00% | 5,041.84 g |

The powder flowed well and no sticking to the logos in the punch occurred, but on the contrary the dispersion of the core was poorer than in the previous example, it being noted that it took longer for the core to break up in water between 19°C and 21°C. As in the previous example, the compressibility of the tablet was good, and the cores achieved a suitable hardness.

Coated dispersible tablets having the following characteristics were obtained:
Biconvex cylindrical white tablets 9.2 mm in diameter.
Theoretical weight of the core of an individual tablet: 265.36 mg ± 5%
Hardness stroke (maximum-minimum): (90-72 N).
Thickness: 4.08 mm.
Moisture content: 3.50%
Disaggregation between: 80 and 135 seconds (n = 6 tablets).

In conclusion it was proposed that a mixture should be prepared using a 1 to 1 mixture of the two pregelatinised starch commercial products employed, applying a laminar coating giving rise to a 1 % increase in the weight of the core.

### Example 3:

Formula of the tablets prepared using a 1 to 1 mixture of the two aforesaid commercial products of pregelatinised starch and mannitol as sweetener. 3 batches of 19,000 cores each were prepared. The coating gave rise to a weight gain of 1%. The composition of the coating was: hydroxypropylmethylcellulose of viscosity between 2.5 and 17.5 mPas as the water-soluble film-forming agent and polyethyleneglycol 6000 as the plasticiser, yielding:
Hydroxypropylmethylcellulose: 2.41 mg / tablet
Polyethylene glycol 6000: 0.24 mg / tablet

| **COMPONENTS** | **CORE** | **%** | **TOTAL BATCH** |
|---|---|---|---|
| Colloidal silicon dioxide (Aerosil 200) | 4.0 mg | 1.51% | 76.00 g |
| Pregelatinised starch (Sepistab and Starch 1500 - (1:1 mixture)) | 45.0 mg | 16.96% | 855.00 g |
| Mint flavour 90351-51 | 4.0 mg | 1.51% | 76.00 g |
| Microcrystalline cellulose | 121.0 mg | 45.60% | 2,299.00 g |
| Fluoxetin hydrochloride | 22.36 mg | 8.43% | 424.84 g |
| Crospovidone (Kollidon CL) | 30.0 mg | 11.31% | 570.00 g |
| D-Mannitol (C Mannidex 16700) | 20.0 mg | 7.54% | 380.00 g |
| Sodium stearyl fumarate (Pruv) | 7.0 mg | 2.64% | 133.00 g |
| Sodium saccharine | 12.0 mg | 4.52% | 228.00 g |
| TOTAL | 265.36 mg | 100.00% | 5,041.84 g |

Coated dispersible tablets having the following characteristics were obtained:
Theoretical weight of the core of an individual tablet: 265.36 mg ± 5%
Hardness stroke (maximum - minimum): 76 - 37 N
Thickness: 4.08 mm
Moisture content: 3.60%
Disaggregation: 38-30 seconds (n = 6 tablets)

### Example 4:

Formula for the tablets prepared as in Example 3 but with Xylitol as sweetener. 3 batches of 19,000 cores were prepared.

The coating yielded a weight gain of 1%.

The composition of the coating was as in the previous example:

Hydroxypropylmethyl cellulose: 2.41 mg / tablet

Polyethyleneglycol 6000: 0.24 mg / tablet.

| **COMPONENTS** | **CORE** | **%** | **TOTAL BATCH** |
|---|---|---|---|
| Colloidal silicon dioxide (Aerosil 200) | 4.0 mg | 1.51% | 76.00 g |
| Pregelatinised starch (Sepistab and Starch 1500 - (1:1 mixture)) | 45.0 mg | 16.96% | 855.00 g |
| Mint flavour 90351-51 | 4.0 mg | 1.51% | 76.00 g |
| Microcrystalline cellulose | 121.0 mg | 45.60% | 2,299.00 g |
| Fluoxetin hydrochloride | 22.36 mg | 8.43% | 424.84 g |
| Crospovidone (Kollidon CL) | 30.0 mg | 11.31% | 570.00 g |
| Xylitol (Xylitab 300) | 20.0 mg | 7.54% | 380.00 g |
| Sodium stearyl fumarate (Pruv) | 7.0 mg | 2.64% | 133.00 g |
| Sodium saccharine | 12.0 mg | 4.52% | 228.00 g |
| TOTAL | 265.36 mg | 100.00% | 5,041.84 g |

Coated dispersible tablets having the following characteristics were obtained:
Theoretical weight of the core of an individual tablet: 265.36 mg ± 5%
Hardness stroke (maximum - minimum): 80 - 40 N
Thickness: 4.05 mm
Moisture content: 3.80%
Disaggregation: 47-32 seconds (n = 6 tablets)

The preparation of suitable formulations for the preparation of coated dispersible tablets requires an investigation of both the physical and chemical incompatibilities between the active ingredient and the excipients and a search for excipients which make it possible to fulfil the requirements specified by the various pharmacopoeias and ensure stability in the mixture. Thus the process of manufacture which is to be used must be borne in mind, given that the excipients will largely depend on the selected process of manufacture.

The process chosen was that of prior partial mixtures in order to finally produce a mixture of all of them, followed by direct compression and the application of an aqueous laminar coating on the cores.

Likewise an important aspect relates to the satisfactory chemical stability of the fluoxetin hydrochloride presented in the form of coated dispersible tablets and packed in aluminium/aluminium blisters, which ensures safety and certain therapeutic advantages under such strict packaging conditions.

In general, laminar coating of the dispersible tablets provides advantages both for industrial manufacture and for use by the patient. Thus, as far as manufacture is concerned, the forms provided with a laminar coating are found to be less sensitive to mechanical erosion and therefore generate less dust, and therefore risks of possible contamination of the premises and between drugs is ruled out. Likewise it is recognised that the laminar coating helps positioning of the tablets in the primary packaging, mainly in blisters and specifically in aluminium/aluminium. As far as use by patients is concerned, the bright or satin texture provided by the laminar film-forming coating mixture improves the finish, prevents adhesion between the bare tablet and the buccal mucosa and makes swallowing easier. As a result of their characteristics these tablets lose the coating in the stomach and completely disaggregate, the effect being the same as if they were taken dispersed in water.

In developing the formulation the excipients were chosen taking into account the rate of disaggregation and the fineness of the dispersion as critical parameters for dispersible tablets. Likewise a laminar coating was designed which did not interfere with disaggregation and dispersion of the tablet. The properties and quality of the tablets and the process of manufacture depend on the quality and the proportions of the excipients chosen. All these excipients were chosen so that direct compression could take place after partial mixing and final mixing and then application of the coating. The fact that a process of direct compression of the mixture is used makes it possible to incorporate the disaggregating agent in an extragranular form, which benefits its swelling effect and therefore the release of fluoxetin.

## Claims

1. Pharmaceutical composition of fluoxetin comprising a coated core, said core containing fluoxetin or a pharmaceutically acceptable salt thereof as active ingredient, **characterised in that** said core is in dispersible tablet form, with the active ingredient micronised and present in an amount of 8% to 9% by weight based on the total weight of said core, **in that** the moisture content of the core is 3% to 4% by weight based on the total weight of said core, and **in that** the core is coated with a film coating comprising water-soluble film-forming agent and plasticiser.

2. A composition as claimed in claim 1 in which the active ingredient comprises fluoxetin hydrochloride.

3. A composition as claimed in claim 1 or 2 in which the film coating is present in an amount of about 1% by weight, based on the weight of the core.

4. A composition as claimed in any preceding claim, in which the said active ingredient is present in an amount of 8.4% to 8.5% by weight based on the total weight of the core.

5. A composition as claimed in claim 4 in which the amount of said active ingredient is 8.43% by weight.

6. A composition as claimed in any preceding claim, in which the film coating comprises hydroxypropyl cellulose as film-forming agent and polyethylene glycol as plasticising agent.

7. A composition as claimed in claim 6 in which the film coating is derived from an aqueous suspension of said hydroxypropylmethyl cellulose having a viscosity of 2.5 to 17.5 mPas and said polyethylene glycol comprising polyethylene glycol 6000 of average molecular weight.

8. A composition as claimed in any preceding claim, wherein the distribution of particle sizes in the fluoxetin active ingredient is as follows : d(0.1) not less than 5 microns d(0.5) between 33 and 35 microns and d(0.9) not less than 180 microns.

9. A composition as claimed in any preceding claim, wherein the core includes a crospovidone as a disaggregating agent and microcrystalline cellulose as diluent.

10. A composition as claimed in claim 9 wherein the crospovidone comprises polyvinylpyrrolidone.

11. A composition as claimed in claim 10 in which the polyvinylpyrrolidone is present at 11.31% by weight based on the total weight of the core, and the microcrystalline cellulose is present at 45.60% by weight based on the weight of the core.

12. A composition as claimed in any preceding claim, wherein the core contains pregelatinised maize starch as an excipient having diluting, aggregating and disaggregating characteristics, comprising a mixture of two pregelatinised starches having different particle size.

13. A composition as claimed in claim 12 in which the core contains 16.96% by weight of said pregelatinised maize starch based on the total weight of the core, and wherein the total said maize starch is derived from a mixture of 50% by weight of each of the two said pregelatinised starches, based on the total weight of said maize starch.

14. A composition as claimed in claim 12 or 13, wherein in the mixture of two pregelatinised maize starches, one of the starches has a partly pregelatinised structure having a composition of about 15% amylopeptin, about 5% amylose and about 80% physically unchanged maize starch, while the other pregelatinised maize starch has granules with a mean particle size of about 200 microns and its tap density is about 0.7g/ml.

15. A composition as claimed in any preceding claim wherein the core includes sodium stearyl fumarate as lubricant and colloidal silicon dioxide as anti-adhesion agent, the core being supplemented with at least one sweetener and at least one flavouring.

16. A composition as claimed in claim 15, in which the said lubricant is present in an amount of about 2.64% by weight based on the total weight of the core, and the said anti-adhesion agent is present in an amount of about 1.51% by weight based on the total weight of the core.

17. A composition as claimed in any preceding claim wherein an artificial sweetener is present, namely a granulated xylitol.

18. A composition as claimed in claim 17 in which the concentration of the xylitol is about 100% and its mean particle size is about 150 microns.

19. A method of making a pharmaceutical composition as claimed in any preceding claim, **characterised in that** the method includes the following steps:
(a) all components in the formulation are sieved through a mesh sieve whereafter separate partial mixing takes place **in that**:
(b) the micronised active ingredient is mixed with about 70% by weight of the total weight of microcrystalline cellulose for about 10 minutes,
(c) separately, about 30% of the remaining microcrystalline cellulose is mixed with the flavouring for about 10 minutes,
(d) separately the crospovidone, the pregelatinised starch, the colloidal silicon dioxide and the sweetener are mixed together for about 10 minutes,
(e) the three separate mixtures obtained in steps (b) to (d) inclusive are mixed together for about 10 minutes whilst sodium stearyl fumarate is added such that the final moisture content of the mixture is 3% to 4% by weight based on the total weight of the core, and
(f) the core is coated with a film coating comprising water-soluble film forming agent and plasticizer.

20. A method as claimed in claim 19 wherein the mixture is rendered homogeneous by the said mixing steps, and is subsequently formed into the said cores by direct compression using a rotary tableting machine, whereafter the coating film is applied to the cores using a perforated pump coating machine with a suspension forming the film coating at a temperature of about 35°C to 45°C.

21. A method as claimed in claim 19 or 20 wherein the film coating is applied to the cores such that the weight of the film-coating represents about 1% by weight based on the total weight of the core.

## Patentansprüche

1. Pharmazeutische Fluotexin-Zusammensetzung, die einen überzogenen Kern umfasst, wobei der Kern Fluoxetin oder ein pharmazeutisch annehmbares Salz davon als Wirkstoff enthält, **dadurch gekennzeichnet, dass** der Kern in Form einer dispergierbaren Tablette vorliegt, wobei der Wirkstoff mikronisiert und in einer Menge von 8 Gew-% bis 9 Gew.-%, bezogen auf das Gesamtgewicht des Kerns, vorhanden ist, dass der Feuchtigkeitsgehalt des Kerns 3 Gew.-% bis 4 Gew.-% beträgt, bezogen auf das Gesamtgewicht des Kerns, und dass der Kern mit einem Filmüberzug überzogen ist, der einen wasserlöslichen Filmbildner und einen Weichmacher umfasst.

2. Eine Zusammensetzung nach Anspruch 1, in welcher der Wirkstoff Fluoxetinhydrochlorid umfasst.

3. Eine Zusammensetzung nach Anspruch 1 oder 2, in welcher der Filmüberzug in einer Menge von zirka 1 Gew.-% vorhanden ist, bezogen auf das Gewicht des Kerns.

4. Eine Zusammensetzung nach einem der vorangehenden Ansprüche, in welcher der Wirkstoff in einer Menge von 8,4 Gew.-% bis 8,5 Gew.-% vorhanden ist, bezogen auf das Gesamtgewicht des Kerns.

5. Eine Zusammensetzung nach Anspruch 4, in welcher die Menge des Wirkstoffes 8,43 Gew.-% beträgt.

6. Eine Zusammensetzung nach einem der vorangehenden Ansprüche, in welcher der Filmüberzug Hydroxypropylcellulose als Filmbildner und Polyethylenglykol als Weichmacher umfasst.

7. Eine Zusammensetzung nach Anspruch 6, in welcher der Filmüberzug aus einer wässerigen Suspension der Hydroxypropylmethylcellulose abgeleitet ist, die eine Viskosität von 2,5 bis 17,5 mPas hat, und das Polyethylenglykol Polyethylenglykol 6000 mit durchschnittlichem Molekulargewicht umfasst.

8. Eine Zusammensetzung nach einem der vorangehenden Ansprüche, wobei die Verteilung der Teilchengrößen in dem Wirkstoff Fluoxetin wie folgt ist: d(0,1) nicht weniger als 5 Mikron, d(0,5) zwischen 33 und 35 Mikron und d(0,9) nicht weniger als 180 Mikron.

9. Eine Zusammensetzung nach einem der vorangehenden Ansprüche, wobei der Kern ein Crospovidon als ein Sprengmittel und mikrokristalline Cellulose als Verdünnungsmittel einschließt.

10. Eine Zusammensetzung nach Anspruch 9, wobei das Crospovidon Polyvinylpyrrolidon umfasst.

11. Eine Zusammensetzung nach Anspruch 10, in welcher das Polyvinylpyrrolidon zu 11,31 Gew.-% vorhanden ist, bezogen auf das Gesamtgewicht des Kerns, und die mikrokristalline Cellulose zu 45,60 Gew.-% vorhanden ist, bezogen auf das Gewicht des Kerns.

12. Eine Zusammensetzung nach einem der vorangehenden Ansprüche, wobei der Kern vorgelatinierte Maisstärke als einen Arzneiträger mit verdünnenden, Binde- und Sprengeigenschaften enthält, die ein Gemisch von zwei vorgelatinierten Stärken mit unterschiedlicher Teilchengröße umfasst.

13. Eine Zusammensetzung nach Anspruch 12, in welcher der Kern zu 16,96 Gew.-% die vorgelatinierte Maisstärke enthält, bezogen auf das Gesamtgewicht des Kerns, und wobei die gesamte Maisstärke aus einem Gemisch von 50 Gew.-% jeder der beiden vorgelatinierten Stärken abgeleitet ist, bezogen auf das Gesamtgewicht der Maisstärke.

14. Eine Zusammensetzung nach Anspruch 12 oder 13, wobei in dem Gemisch von zwei vorgelatinierten Maisstärken eine der Stärken eine teilweise vorgelatinierte Struktur hat, die eine Zusammensetzung von zirka 15 % Amylopeptin, zirka 5 % Amylose und zirka 80 % materiell unveränderter Maisstärke hat, während die andere vorgelatinierte Maisstärke ein Granulat mit einer mittleren Teilchengröße von zirka 200 Mikron und einer Klopfdichte von zirka 0.7 g/ml ist.

15. Eine Zusammensetzung nach einem der vorangehenden Ansprüche, wobei der Kern Natriumstearylfumarat als Gleitmittel und kolloidales Siliciumdioxid als Antihaftmittel einschließt, wobei der Kern mit mindestens einem Süßungsmittel und mindestens einem Geschmacksstoff ergänzt ist.

16. Eine Zusammensetzung nach Anspruch 15, in welcher das Gleitmittel in einer Menge von zirka 2,64 Gew.-%, bezogen auf das Gesamtgewicht des Kerns, vorhanden ist und das Antihaftmittel in einer Menge von zirka 1,51 Gew.-%, bezogen auf das Gesamtgewicht des Kerns, vorhanden ist.

17. Eine Zusammensetzung nach einem der vorangehenden Ansprüche, wobei ein künstliches Süßungsmittel vorhanden ist, nämlich ein granuliertes Xylitol.

18. Eine Zusammensetzung nach Anspruch 17, in welcher die Konzentration des Xylitols zirka 100 % beträgt und seine mittlere Teilchengröße zirka 150 Mikron beträgt.

19. Ein Verfahren zur Herstellung einer pharmazeutischen Zusammensetzung nach einem der vorangehenden Ansprüche, **dadurch gekennzeichnet, dass** das Verfahren die folgenden Schritte einschließt:
(a) alle Komponenten in der Rezeptur werden durch ein Maschen-Sieb gesiebt, wonach getrenntes, teilweises Mischen stattfindet, indem:
(b) der mikronisierte Wirkstoff mit zirka 70 Gew.-% der mikrokristallinen Cellulose, bezogen auf deren Gesamtgewicht, zirka 10 Minuten lang gemischt wird,
(c) davon getrennt, zirka 30 % der verbleibenden mikrokristallinen Cellulose zirka 10 Minuten lang mit dem Geschmacksstoff gemischt werden,
(d) davon getrennt, das Crospovidon, die vorgelatinierte Stärke, das kolloidale Siliciumdioxid und das Süßungsmittel zirka 10 Minuten lang zusammengemischt werden,
(e) die drei getrennten, in den Schritten (b) bis einschließlich (d) erhaltenen Gemische zirka 10 Minuten lang zusammengemischt werden, während Natriumstearylfumarat hinzugefügt wird, sodass der Feuchtigkeitsgehalt des Gemisches schließlich 3 Gew.-% bis 4 Gew.-%, bezogen auf das Gesamtgewicht des Kerns, beträgt, und
(f) der Kern mit einem Filmüberzug überzogen wird, der einen wasserlöslichen Filmbildner und einen Weichmacher umfasst.

20. Ein Verfahren nach Anspruch 19, wobei das Gemisch durch die Mischungsschritte homogen gemacht wird und anschließend mittels einer Rundläufertablettiermaschine durch direkte Komprimierung zu den Kernen geformt wird, wonach der Filmüberzug mittels einer Beschichtungsmaschine mit Lochpumpe auf die Kerne mit einer Suspension aufgetragen wird, die den Filmüberzug bei einer Temperatur von zirka 35° C bis 45° C bildet.

21. Ein Verfahren nach Anspruch 19 oder 20, wobei der Filmüberzug so auf die Kerne aufgetragen wird, dass das Gewicht des Filmüberzuges zirka 1 Gew.-%, bezogen auf das Gesamtgewicht des Kerns, ausmacht.

## Revendications

1. Composition pharmaceutique à base de fluoxétine comprenant un noyau enrobé, ledit noyau contenant de la fluoxétine ou un sel pharmaceutiquement acceptable de celle-ci comme principe actif, **caractérisée en ce que** ledit noyau se présente sous forme de comprimé dispersible, le principe actif étant micronisé et présent dans une quantité allant de 8 % à 9 % en poids sur la base du poids total dudit noyau, **en ce que** la teneur en humidité du noyau est de 3 % à 4 % en poids sur la base du poids total dudit noyau et **en ce que** le noyau est enrobé d'un enrobage pelliculé comprenant un agent filmogène hydrosoluble et un plastifiant.

2. Composition selon la revendication 1, dans laquelle le principe actif comprend du chlorhydrate de fluoxétine.

3. Composition selon la revendication 1 ou 2, dans laquelle l'enrobage pelliculé est présent dans une quantité d'environ 1 % en poids sur la base du poids du noyau.

4. Composition selon l'une quelconque des revendications précédentes, dans laquelle ledit principe actif est présent dans une quantité de 8,4 % à 8,5 % en poids sur la base du poids total du noyau.

5. Composition selon la revendication 4, dans laquelle la quantité dudit principe actif est de 8,43 % en poids.

6. Composition selon l'une quelconque des revendications précédentes, dans laquelle l'enrobage pelliculé comprend de l'hydroxypropyl cellulose comme agent filmogène et du polyéthylène glycol comme agent plastifiant.

7. Composition selon la revendication 6, dans laquelle l'enrobage pelliculé est dérivé d'une suspension aqueuse à base de ladite hydroxypropylméthyl cellulose ayant une viscosité de 2,5 à 17,5 mPas et ledit polyéthylène glycol comprenant du polyéthylène glycol 6000 de masse moléculaire moyenne.

8. Composition selon l'une quelconque des revendications précédentes, dans laquelle la répartition des tailles de particules dans le principe actif fluoxétine est la suivante : d(0,1) d'au moins 5 microns, d(0,5) comprise entre 33 et 35 microns et d(0,9) d'au moins 180 microns.

9. composition selon l'une quelconque des revendications précédentes, dans laquelle le noyau comprend une crospovidone comme agent délitant et de la cellulose microcristalline comme diluant.

10. Composition selon la revendication 9, dans laquelle la crospovidone comprend de la polyvinylpyrrolidone.

11. Composition selon la revendication 10, dans laquelle la polyvinylpyrrolidone est présente à 11,31 % en poids sur la base du poids total du noyau et la cellulose microcristalline est présente à 45,60 % en poids sur la base du poids du noyau.

12. Composition selon l'une quelconque des revendications précédentes, dans laquelle le noyau contient de l'amidon de maïs prégélatinisé comme excipient ayant des caractéristiques de dilution, d'agrégation et de délitement, comprenant un mélange de deux amidons prégélatinisés ayant différents tailles de particules.

13. Composition selon la revendication 12, dans laquelle le noyau contient 16,96 % en poids dudit amidon de maïs prégélatinisé sur la base du poids total du noyau et dans laquelle la totalité de l'amidon de maïs est dérivée d'un mélange composé de 50 % en poids de chacun des deux dits amidons prégélatinisés, sur la base du poids total dudit amidon de maïs.

14. Composition selon la revendication 12 ou 13, dans laquelle dans le mélange de deux amidons de maïs prégélatinisés, l'un des amidons a une structure partiellement prégélatinisée ayant une composition d'environ 15 % d'amylopeptine, d'environ 5 % d'amylose et d'environ 80 % d'amidon de maïs physiquement intact, tandis que l'autre amidon de maïs prégélatinisé a des granules ayant une taille de particules moyenne d'environ 200 microns et sa masse volumique après tassement est d'environ 0,7 g/ml.

15. Composition selon l'une quelconque des revendications précédentes, dans laquelle le noyau comprend du stéarylfumarate de sodium comme lubrifiant et de la silice colloïdale comme agent antiadhésif, le noyau étant supplémenté avec au moins un édulcorant et au moins un arôme.

16. Composition selon la revendication 15, dans laquelle ledit lubrifiant est présent dans une quantité d'environ 2,64 % en poids sur la base du poids total du noyau et ledit agent antiadhésif est présent dans une quantité d'environ 1,51 % en poids sur la base du poids total du noyau.

17. Composition selon l'une quelconque des revendications précédentes, dans laquelle un édulcorant artificiel est présent, à savoir, un xylitol sous forme de granules.

18. Composition selon la revendication 17, dans laquelle la concentration du xylitol est d'environ 100 % et sa taille de particules moyenne est d'environ 150 microns.

19. Procédé de fabrication d'une composition pharmaceutique selon l'une quelconque des revendications précédentes, **caractérisé en ce que** le procédé comprend les étapes suivantes :
(a) tous les composants dans la formulation sont criblés à travers un crible à mailles, après quoi un mélange partiel distinct s'opère **en ce que** :
(b) le principe actif micronisé est mélangé avec environ 70 % en poids du poids total de la cellulose microcristalline pendant environ 10 minutes,
(c) séparément, on mélange pendant environ 10 minutes environ 30 % de la cellulose microcristalline restante avec l'arôme,
(d) séparément, on mélange pendant environ 10 minutes la crospovidone, l'amidon prégélatinisé, la silice colloïdale et l'édulcorant,
(e) les trois mélanges distincts obtenus aux étapes (b) à (d) incluse sont mélangés pendant environ 10 minutes tandis que l'on ajoute du stéarylfumarate de sodium, de telle sorte que la teneur finale en humidité du mélange soit de 3 % à 4 % en poids sur la base du poids total du noyau, et
(f) le noyau est enrobé d'un enrobage pelliculé comprenant un agent filmogène hydrosoluble et un plastifiant.

20. Procédé selon la revendication 19, dans lequel on rend le mélange homogène par lesdites étapes de mélange et il est ensuite formé pour donner lesdits noyaux par compression directe en utilisant une machine rotative pour la fabrication des comprimés, après quoi la pellicule d'enrobage est appliquée sur les noyaux en utilisant une enrobeuse à pompe perforée avec une suspension formant l'enrobage pelliculé à une température d'environ 35 °C à 45 °C.

21. Procédé selon la revendication 19 ou 20, dans lequel l'enrobage pelliculé est appliqué sur les noyaux de manière à ce que le poids de l'enrobage pelliculé représente environ 1 % en poids sur la base du poids total du noyau.
